# EUROPÄISCHE PATENTANMELDUNG

(11) **EP 2 674 142 A1**
(43) Veröffentlichungstag der Anmeldung: **18.12.2013**
(21) Anmeldenummer: 12172221.9
(22) Anmeldetag: 15.06.2012
(51) Int. Cl.: A61F 13/20, A61H 23/04

(54) **Tamponanordnung, Verwendung einer Tamponanordnung, Verfahren und Verwendung eines Druckgasbehälters**

(71) Anmelder: Marlafin AG, 6332 Hagendorn (CH)
(72) Erfinder: Mock, Elmar, 2013 Colombier (CH); Sigrist, Martin, 3008 Bern (CH); Bitmead, Naomi, 3047 Bremgarten bei Bern (CH); Aeschlimann, Marcel, 2514 Ligerz (CH); Wehren, Salome, 2503 Biel (CH)
(74) Vertreter: AMMANN PATENTANWÄLTE AG BERN

(57) **Zusammenfassung**

Die Tamponanordnung (1) umfasst einen Saugkörper (2) und einen damit verbundenen Vibrationserzeuger (3). Die Tamponanordnung (1) weist einen Strömungserzeuger (8) für ein Fluid auf, welcher über eine Fluidleitung (5) mit dem Vibrationserzeuger (3) wirkverbunden oder wirkverbindbar ist. Damit zum Betrieb des Vibrationserzeugers (3) kein elektrischer Strom zum Saugkörper (2) geleitet werden muss, kann die Tamponanordnung (1) eine Fluiddruckquelle (7, 8) enthalten. Zudem betrifft die Erfindung eine Verwendung einer Tamponanordnung (1), ein Verfahren zum Aufsaugen von Körperflüssigkeiten in einer Vagina und/oder zur Schwingungsbeaufschlagung eines weiblichen Körpers im Bereich der Vagina sowie eine Verwendung eines Druckbehälters (7).

## Beschreibung

Die Erfindung betrifft eine Tamponanordnung mit einem Saugkörper und einem damit verbundenen Vibrationserzeuger. Zudem betrifft die Erfindung eine Verwendung einer Tamponanordnung, ein Verfahren zum Aufsaugen von Körperflüssigkeiten in einer Vagina und/oder zur Schwingungsbeaufschlagung eines weiblichen Körpers im Bereich der Vagina sowie eine Verwendung eines Druckbehälters.

Vibrierende Tamponanordnungen sind bekannt, beispielsweise aus dem Dokument EP 1231885 B1. Diese Anordnung dient zur Bekämpfung von menstrualen Schmerzen und umfasst einen in einem Tampon angeordneten, elektrisch angetriebenen Vibrationserzeuger und eine mit dem Tampon durch ein Kabel verbundene Energieversorgungseinheit, in welcher eine Energiequelle und ein als Drucktaste ausgebildetes Bedienungselement angeordnet ist, das die elektrische Verbindung zwischen der elektrischen Energiequelle und dem Vibrationserzeuger steuert. Wenn auch die zum Betrieb des Vibrationserzeugers verwendete Spannung relativ gering ist und beispielsweise 1,35 Volt beträgt und alle elektrischen Komponenten elektrisch isoliert sind, so haben doch viele potentielle Benutzerinnen Vorbehalte dagegen, elektrischen Strom in das Innere ihres Körpers zu leiten.

Ausgehend von diesem Stand der Technik liegt der Erfindung die Aufgabe zu Grunde, eine Tamponanordnung mit einem mit einem Tampon verbundenen Vibrationserzeuger zur Verfügung zu stellen, bei der im Betrieb kein elektrischer Strom zum Tampon geleitet wird.

Diese Aufgabe wird erfindungsgemäss dadurch gelöst, dass die Tamponanordnung einen Strömungserzeuger für ein Fluid aufweist, welcher über eine Fluidleitung mit dem Vibrationserzeuger wirkverbunden oder wirkverbindbar ist. Durch Druckänderung und/oder eine Fluidströmung in der Fluidleitung können der Vibrationserzeuger und damit auch der Saugkörper zu Vibrationen angeregt werden.

Durch die Verwendung eines Fluids als Energietransportmittel kann der Vibrationserzeuger ohne elektrischen Strom betrieben werden. Je nach Ausgestaltung der das Fluid führenden Komponenten der Anordnung kann das Fluid ein Gas oder eine Flüssigkeit sein. Unter einem Strömungserzeuger wird ein jedes Mittel verstanden, welches dazu geeignet ist, eine Strömung in dem Fluid zu erzeugen. So kann es sich bei dem Strömungserzeuger um eine Pumpe, insbesondere eine Pumpe mit umkehrbarer Saug- und Druckrichtung, ein Gebläse, einen Verdichter, eine Turbine etc. handeln. Ganz allgemein betrachtet kann es sich bei dem Strömungserzeuger somit um eine Fluidenergiemaschine und/oder einen wie weiter unten beschriebenen Fluiddruckerzeuger handeln.

Die Tamponanordnung kann den Strömungserzeuger enthalten - optional als vom Saugkörper getrenntes Bauteil. Die Erfindung umfasst jedoch auch Tamponanordnungen, welche den Strömungserzeuger nicht enthalten, sondern deren Vibrationserzeuger lediglich über die Fluidleitung mit einem Strömungserzeuger wirkverbindbar ist.

Die Fluidleitung weist bevorzugt eine Länge mindestens 20 mm, bevorzugt mindestens 50 mm auf. Eine solche Länge erlaubt es, den Strömungserzeuger bzw. die den Strömungserzeuger enthaltende Energieversorgungseinheit ausserhalb des Körpers der die Tamponanordnung tragenden Person anzuordnen.

Um eine gute Handhabbarkeit zu ermöglichen und einen grösstmöglichen Tragekomfort zu gewährleisten, kann es nach einer bevorzugten Variante der Erfindung vorgesehen sein, dass der Strömungserzeuger in einer Energieversorgungseinheit enthalten ist, welche zusammen mit dem Strömungserzeuger eine Baueinheit bildet.

Alternativ können Strömungserzeuger und Energieversorgungseinheit jedoch auch zwei voneinander beabstandete Bauteile sein, die durch eine Leitung miteinander verbindbar oder verbunden sein können. Die Länge dieser Leitung kann mindestens 20 mm, bevorzugt mindestens 50 mm sein.

Eine Ausführungsform der Erfindung, welche einen sehr kompakten Aufbau und einen guten Wirkungsgrad hinsichtlich der Erzeugung einer Fluidströmung aufweist, sieht vor, dass der Strömungserzeuger mit dem Saugkörper und dem Vibrationserzeuger eine Baueinheit bildet. Eine Energieversorgungseinheit kann in einer Distanz von mindestens 20 mm vom Saugkörper angeordnet sein.

Gemäss einer Ausführungsform der Erfindung, welche sich durch einen sehr zuverlässigen Betrieb und einen einfachen Aufbau auszeichnet, kann der Strömungserzeuger einen Fluiddruckerzeuger umfassen. Entsprechend einer besonders günstigen Weiterbildung dieser Variante, welche sich durch eine besonders einfache Handhabung auszeichnet, kann es vorgesehen sein, dass der Fluiddruckerzeuger einen mit Druckfluid vorbefüllten Druckbehälter, z.B. eine Druckgaspatrone, enthält oder daraus besteht.

Entsprechend einer Weiterbildung dieser Variante der Erfindung kann es auch vorgesehen sein, dass der Fluiddruckerzeuger einen wieder befüllbaren Druckbehälter umfasst. Ein Vorteil dieser Variante besteht darin, dass das Fluid durch Umkehr der Strömungsrichtung wieder in den wieder befüllbaren Druckbehälter zurückströmen kann.

Ein modularer Aufbau, der einen einfachen und schnellen Austausch einzelner Komponenten ermöglicht, lässt sich dadurch realisieren, dass der Fluiddruckerzeuger und/oder der Druckbehälter über eine Schnellverschlusskupplung mit dem Strömungserzeuger und/oder mit dem Vibrationserzeuger kuppelbar sind.

Um zu hohe Drücke zu vermeiden und eine grösstmögliche Sicherheit bei der Benutzung zu gewährleisten, kann es vorgesehen sein, dass zwischen dem Fluiddruckerzeuger und dem Druckbehälter ein bevorzugt einstellbares Druckreduzier- und/oder Überdruckventil angeordnet ist.

Eine vorteilhafte Variante der Erfindung besteht darin, dass am Vibrationserzeuger eine weitere Fluidleitung angeschlossen ist. Diese Variante der Erfindung ermöglicht beispielsweise die Realisierung eines geschlossenen Fluidkreislaufes: Das Fluid kann durch die erste, erfindungsgemässe Fluidleitung in den Vibrationserzeuger einströmen und durch die weitere Fluidleitung wieder aus diesem herausströmen.

Ein einfacher Austausch der Fluidleitung lässt sich dadurch erreichen, dass die Fluidleitung an dem mit dem Strömungserzeuger verbundenen Ende und/oder an dem mit dem Vibrationserzeuger verbundenen Ende mit lösbaren Kupplungsmitteln ausgestattet ist.

Eine Variante der Erfindung, welche sich ebenfalls durch einen einfachen Aufbau auszeichnet, sieht vor, dass der Vibrationserzeuger mindestens einen durch einen Fluidstrom in eine rotierende Bewegung versetzbaren Körper enthält. Hierbei hat es sich als vorteilhaft erwiesen, wenn Führungsmittel zum Führen des Körpers auf einer Umlaufbahn vorgesehen sind.

Alternativ zu der zuletzt genannten Variante kann es jedoch auch von Vorteil sein, wenn der Körper rotierbar auf einer Achse oder Welle angeordnet ist. Dies ist insbesondere dann von Vorteil, wenn die Welle mit einer Turbine verbunden ist.

Um eine zuverlässige Vibration zu gewährleisten, kann es vorgesehen sein, dass der Vibrationserzeuger mindestens einen durch komprimiertes Fluid in eine oszillierende Bewegung versetzbaren Körper enthält. Als günstig hat es sich diesbezüglich herausgestellt, wenn der Körper als in einem Zylinder geführter Kolben oder als Lamelle ausgebildet ist.

Gemäss einer weiteren Variante der Erfindung kann es vorgesehen sein, dass der Vibrationserzeuger einen mit Fluiddruck beaufschlagbaren, elastischen Hohlkörper enthält.

Um ein einfaches Entfernen von im Körper befindlichen Teilen der Tamponanordnung zu gewährleisten, kann es vorgesehen sein, dass am Vibrationserzeuger und/oder am Saugkörper zumindest ein bevorzugt flexibles Entnahmeorgan, insbesondere ein Zugelement wie ein Faden oder eine Schnur, befestigt ist.

Ein einfaches Austauschen des Fluiddruckerzeugers bzw. des Druckbehälters lässt sich dadurch bewerkstelligen, dass der Fluiddruckerzeuger und/oder der Druckbehälter über eine Schnellverschlusskupplung mit dem Strömungserzeuger und/oder mit dem Vibrationserzeuger kuppelbar sind.

Um eine einfache Austauschbarkeit des Saugkörpers bzw. Vibrationserzeugers und eine Wiederverwendbarkeit der anderen Komponenten zu ermöglichen, kann es vorgesehen sein, dass der Saugkörper und der Vibrationserzeuger und/oder der Strömungserzeuger mit zusammenwirkenden Kupplungsteilen versehen sind.

Um einen Kontakt der Kupplungsstelle des Saugkörpers mit dem Körperinneren zu vermeiden, kann es vorgesehen sein, dass der Kupplungsteil des Saugkörpers in einem vom Saugkörper zumindest zum Teil, bevorzugt vollständig umschlossenen Hohlraum angeordnet ist.

Ein weiterer Aspekt der Erfindung betrifft die Verwendung einer wie oben beschriebenen Tamponanordnung zum Aufsaugen von Körperflüssigkeiten in einer Vagina und/oder zur Schwingungsbeaufschlagung eines weiblichen Körpers im Bereich der Vagina. Erfindungsgemäss wird der mit dem Vibrationserzeuger verbundene Saugkörper in die Vagina eingesetzt. Der Strömungserzeuger kann lösbar auf der Körperoberfläche und/oder an einem Kleidungsstück befestigt werden. Die Befestigung auf der Körperoberfläche kann beispielsweise mittels eines Pflasters erfolgen. Bei dem Kleidungsstück kann es sich etwa um einen Slip handeln. Alternativ dazu kann die Befestigung auch an einer Slipeinlage der Benutzerin erfolgen.

Noch ein weiterer Aspekt der Erfindung betrifft ein Verfahren zum Aufsaugen von Körperflüssigkeiten in einer Vagina und/oder zur Schwingungsbeaufschlagung eines weiblichen Körpers im Bereich der Vagina mit einem in die Vagina eingesetzten Saugkörper. Der Vibrationserzeuger wird mit einem Strömungserzeuger und optional auch mit einer Energiequelle verbunden. Der Vibrationserzeuger kann zumindest intermittierend vom Strömungserzeuger zu Vibrationen beaufschlagt werden. Nach Ablauf einer vorbestimmbaren Zeitdauer kann der Saugkörper mit dem Vibrationserzeuger entfernt und entsorgt werden. Durch Druckänderung und/oder eine Fluidströmung können der Vibrationserzeuger und damit auch der Saugkörper zu Schwingungen angeregt werden.

Zudem umfasst die Erfindung auch die Verwendung eines Druckbehälters, insbesondere einer Druckgaspatrone, als Strömungserzeuger in einer Tamponanordnung, insbesondere in einer wie oben beschriebenen Tamponanordnung.

Besondere Ausführungsarten der Erfindung sind in den abhängigen Ansprüchen umschrieben.

Ausführungsbeispiele der Erfindung werden nachstehend unter Bezugnahme auf die angefügten Zeichnungen beispielsweise näher beschrieben. Es zeigen schematisch
- Figur 1: eine Darstellung eines Ausführungsbeispiels der erfindungsgemässen Tamponanordnung;
- Figur 2: den Tampon entsprechend Figur 1 in einer vergrösserten Darstellung;
- Figur 3: eine weitere Möglichkeit der Anordnung des Vibrationserzeugers im Tampon;
- Figur 4: eine weitere Möglichkeit der Anordnung des Vibrationserzeugers im Tampon;
- Figur 5: eine Ausführungsart des Vibrationserzeugers;
- Figuren 6-13: je eine weitere Ausführungsart des Vibrationserzeugers;
- Figur 14: eine Darstellung eines weiteren Ausführungsbeispiels der erfindungsgemässen Tamponanordnung;
- Figur 15: eine Ausführungsart eines Vibrationserzeugers, insbesondere zur Verwendung mit einer Tamponanordnung nach Figur 14;
- Figuren 16-20: je eine weitere Ausführungsart eines Vibrationserzeugers, insbesondere zur Verwendung mit einer Tamponanordnung nach Figur 14;
- Figur 21: eine weitere Ausführungsform eines Saugkörpers einer erfindungsgemässen Tamponanordnung in Seitenansicht geschnitten;
- Figur 22: eine andere Ausbildung einer Tamponanordnung mit einem Vibrationserzeuger in Seitenansicht, teilweise geschnitten gemäss den Pfeilen XXII-XXII in Figur 23;
- Figur 23: die Tamponanordnung nach Figur 22 in Draufsicht geschnitten, gemäss den Linien XXIII-XXIII in Figur 22;
- Figur 24: eine weitere Ausbildung eines Saugkörpers aus Schaumstoff in Seitenansicht, teilweise geschnitten;
- Figur 25: die Anordnung einer Tamponanordnung 1 mit dem in der Vagina befindlichen Saugkörper und Energiequelle 7 auf einem Bekleidungsstück der Benutzerin in stark vereinfachter schematischer Darstellung.

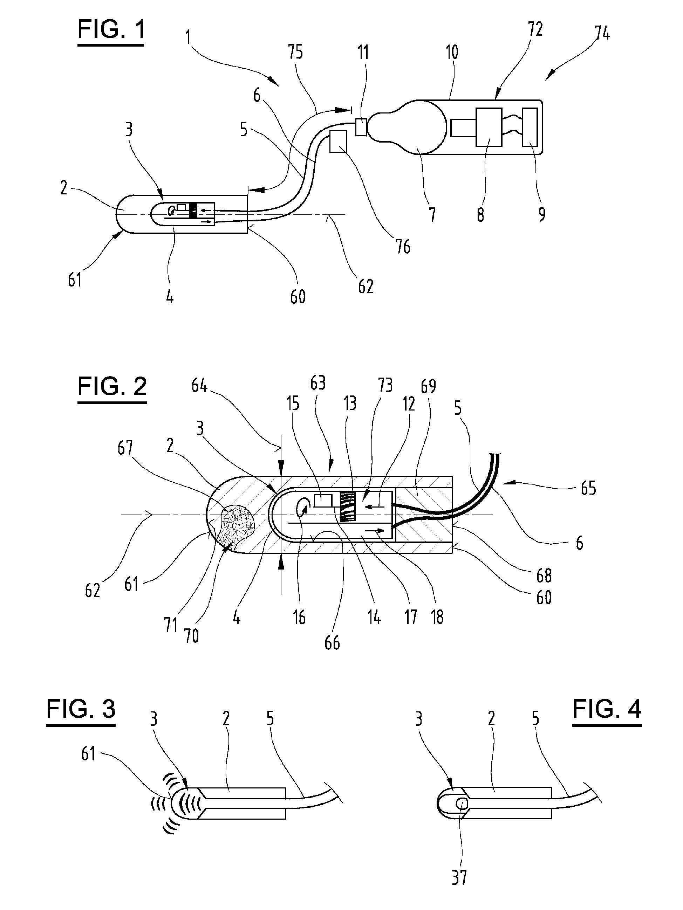

Figur 1 zeigt in einer schematischen Darstellung ein Ausführungsbeispiel einer erfindungsgemässen Tamponanordnung 1. Die Anordnung umfasst einen tamponartigen Saugkörper 2 aus saugfähigem Material und einen Vibrationserzeuger 3, der in einem Gehäuse 4 angeordnet ist.

Im Folgenden soll kurz auf den Aufbau der Tamponanordnung 1 eingegangen werden, wobei diese nachstehenden Ausführungen zum Aufbau der erfindungsgemässen Tamponanordnung 1 und die darin genannten Merkmale einzeln oder in beliebiger Kombination mit den verschiedenen Ausführungen und den Merkmalen in den nachfolgenden Ausführungsbeispielen kombiniert werden können.

Der vorliegende Tampon, bzw. der tamponartige Saugkörper 2 enthält ein proximales Ende 60 und ein distales Ende 61. Am proximalen Ende 60 kann ein flexibles Entnahmeorgan 65, z.B. ein Faden, eine Schnur oder eine flexible Verbindung angeordnet sein. Insbesondere kann das Entnahmeorgan 65 auch durch die weiter unten beschriebene Fluidleitung 5 selbst gebildet sein. Der Begriff "proximales Ende" 60, wie er hierin verwendet wird, bezieht sich auf jene Abschnitte der Anordnung und ihre Teile, die von dem Körper einer Benutzerin am weitesten entfernt sind, wenn der tamponartige Saugkörper 2 bestimmungsgemäss in eine Körperöffnung, z.B. die Vagina, eingeführt wird. Der Begriff "distales Ende" 61 bezieht sich auf jene Abschnitte der Anordnung und ihre Teile, die dem Körper einer Benutzerin am nächsten sind, wenn der Saugkörper 2 eingeführt wird. Demzufolge geben die Begriffe "proximal" oder "distal", wie sie hierin verwendet werden, an, dass ein bestimmter Teil oder eine bestimmte Struktur der Anordnung oder ihre Teile dem proximalen Ende 60 bzw. dem distalen Ende 61 der Anordnung oder ihrer Teile näher ist. In ähnlicher Art und Weise beziehen sich die Begriffe "proximale Richtung" oder "distale Richtung" auf die Richtungen zum proximalen Ende 60 bzw. zum distalen Ende 61 des tamponartigen Saugkörpers 2 hin.

Der tamponartige Saugkörper 2 besitzt einen länglichen Hauptteil, der ein distales Einschubende, ein proximales Rückzugsende und einen mittleren Teil 63 bildet, der dazwischen verläuft. Der tamponartige Saugkörper 2 besitzt ausserdem eine Längsachse 62 und eine Aussenseite aus einem saugenden bzw. hygroskopischen Material. Der tamponartige Saugkörper 2 oder zumindest der mittlere Teil 63 des Saugkörpers 2 kann eine im Wesentlichen zylindrische bzw. hohlzylindrische Form aufweisen, wobei es sich um eine einfache geometrische Form einer zylindrischen Hülle handeln kann, die der Gesamtform des tamponartigen Saugkörpers 2 oder zumindest ihres mittleren Teils 63 am nächsten kommt. Der tamponartige Saugkörper 2 kann an seiner Aussenseite auch Rippen und Rillen aufweisen oder an seiner Aussenseite im Wesentlichen glatt ausgebildet sein.

Der maximale Aussendurchmesser 64 des tamponartigen Saugkörper 2 oder seines mittleren Teiles 63 kann in Längsrichtung im Wesentlichen gleichmässig sein oder er kann variieren. Der Teil des tamponartigen Saugkörpers 2 nahe an dem proximalen Ende 60 kann beispielsweise einen grösseren maximalen Aussendurchmesser besitzen als im mittleren Teil 63. Durch diese Verdickung am proximalen Ende 60 des tamponartigen Saugkörpers 2 verringert sich die Gefahr, dass Körperflüssigkeit austritt, wenn der tamponartige Saugkörper 2 in der Vagina platziert wird.

Der tamponartige Saugkörper 2 unterscheidet sich von den herkömmlichen Tampons, da die bekannten Tampons aus einem Vollkörper aus saugfähigem Material bestehen. Zudem ist das saugfähige Material, insbesondere im Bereich von Rillen oder Vertiefungen, stark verdichtet.

Die tamponartigen Saugkörper 2, die in Verbindung mit einem Vibrationserzeuger 3 stehen, weisen demgegenüber im Inneren einen Hohlraum 66 auf. Dementsprechend ist der Saugkörper 2 haubenförmig bzw. hohlzylinderförmig, wobei zumindest eines der beiden Stirnenden des Hohlzylinders bevorzugt im Bereich des distalen Endes 61 mit einer Stirnwand 67 verschlossen ist. Weiterhin kann das gegenüberliegende stirnseitige proximale Ende 60 offen oder ebenfalls mit einer Stirnwand 68 verschlossen sein, die z.B. durch einen Einsatzteil 69, wie einen Stöpsel, gebildet sein kann. Alternativ kann in der Stirnwand 68 eine bevorzugt öffen- bzw. ausdehnbare und verschliessbare Öffnung angeordnet sein.

Das Ausmass der Verdichtung des tamponförmigen Saugkörpers 2 und damit die Masse an Fasern 70 und Fäden 71, an welchen die Flüssigkeit anhaften kann, kann entsprechend den verschiedenen Ausführungen des Vibrationserzeugers 3 erfolgen. So kann der hohlzylindrische, tamponartige Saugkörper 2 wie bei der Ausführungsform in den Figuren 1 und 2 eine sehr hohe Eigensteifigkeit gegen Verbiegungen quer zur Längsachse 62 aufweisen. Dadurch kann zweckmässig erreicht werden, dass die Vibrationen besser, z.B. mit geringerer Dämpfung, auf den Körper übertragen werden.

Es kann andererseits aber bei Vibrationserzeugern 3, die durch ihr Volumen ändernde Körper gebildet sind, der Saugkörper 2 zumindest eine Elastizität für Dehnung in radialer Richtung aufweisen. Bevorzugt kann hierzu der mittlere Teil 63 zwischen den beiden Stirnwänden 67, 68 mit Schwächungen oder Faltenbildungen im hohlzylindrischen Mantel ausgebildet sein, die in Längsrichtung, das heisst parallel zur Längsachse 62 des Hohlzylinders, als Rillen oder Einschnitte verlaufen können. Gemäss einer anderen Ausführungsvariante ist es aber ebenso möglich, dass die Schwächungsbereiche als radial umlaufende Zonen in einer zur Längsachse 62 des Saugkörpers 2 quer oder schräg verlaufenden Ebene ausgebildet sind.

Nach einer anderen Weiterbildung ist es aber auch möglich, den Saugkörper 2 so auszubilden, dass er bei einer Verformung elastisch selbstrückstellend wirkt. Dazu können in dem Material des Saugkörpers elastische Fäden, die in Längsrichtung und/oder Querrichtung zur Längsachse 62 des Saugkörpers 2 verlaufen, eingearbeitet oder in auf der den Hohlraum 66 begrenzenden Innenwand des Saugkörpers 2 eingebracht oder aufgebracht sein. Beispielsweise ist es aber auch möglich, den Saugkörper 2 aussen mit einer in radialer Richtung vorgespannten, elastisch verformbaren und selbstrückstellenden Umhüllung oder Haube oder Ringen zu versehen.

Zur Herstellung der Einheit aus Vibrationserzeuger 3 und tamponartigem Saugkörper 2 kann der Vibrationserzeuger 3 mit einem saugfähigen Fasermaterial umwickelt bzw. umgeben werden, welches den tamponartigen Saugkörper 2 bildet. Der Vibrationserzeuger 3 kann beispielsweise in das Material des Saugkörpers 2 eingenäht sein oder aber auch durch eingangs genannte Rückhaltelemente an einem Herausrutschen aus dem Saugkörper 2 gehindert werden. Alternativ sind jedoch noch eine grosse Anzahl anderer Massnahmen zur Verbindung des Vibrationserzeugers 3 mit dem tamponartigen Saugkörper 2 möglich. So könnte nach einem Umwickeln des Vibrationserzeugers 3 mit dem tamponartigen Saugkörper 2 das proximale Ende 60 des Saugkörpers 2 dicht gepresst werden, sodass dieser gepresste Abschnitt ein Herausziehen des Vibrationserzeugers 3 verhindert.

Saugfähiges Fasermaterial für den tamponartigen Saugkörper 2 kann aus jedem beliebigen, saugfähigen Material bestehen, das akzeptable Saugfähigkeitseigenschaft und Elastizitätsmoduleigenschaften besitzt, welche Flüssigkeit aufnehmen und/oder zurückhalten können. Die saugfähige Struktur lässt sich aus einer Vielzahl von Grössen und Formen und aus einer Vielzahl von flüssigkeitsabsorbierenden Materialien herstellen. Selbstverständlich ist es wünschenswert, saugfähige Materialien zu verwenden, die einen Mindestgehalt an fremdlöslichen Materialien enthalten, da das Produkt für einen bestimmten Zeitraum im Körper verbleibt. Zurückgehaltene lösliche Fremdmaterialien könnten ein Sicherheitsrisiko darstellen, wenn sie toxisch, reizend oder empfindlich sind.

Eine repräsentative, nicht einschränkend zu verstehende Liste brauchbarer Materialien enthält zellulosehaltige Materialien wie beispielsweise Rayon, Baumwolle, Zellstoff, Zellstoffwatte, Tissue, Tissue-Schichtstoffe, Torfmull und chemisch verstärkte, modifizierte oder vernetzte Zellulosefasern; synthetische Materialien wie beispielsweise Polyesterfasern, Polyolefinfasern, saugfähige Schaumstoffe, beispielsweise einen elastisch federnden Polyurethanschaumstoff, saugfähige Schwämme, äusserst saugfähige Polymere, saugfähige, gelbildende Materialien; bearbeitete Fasern wie beispielsweise kapillare Kanalfasern und Fasern mit mehreren Gliedern; synthetische Fasern oder ein äquivalentes Material oder Kombinationen von Materialien oder Mischungen daraus.

Der Saugkörper 2 kann aber auch aus einem Kunststoffschaum, zum Beispiel einem Polyurethanschaum, hergestellt sein.

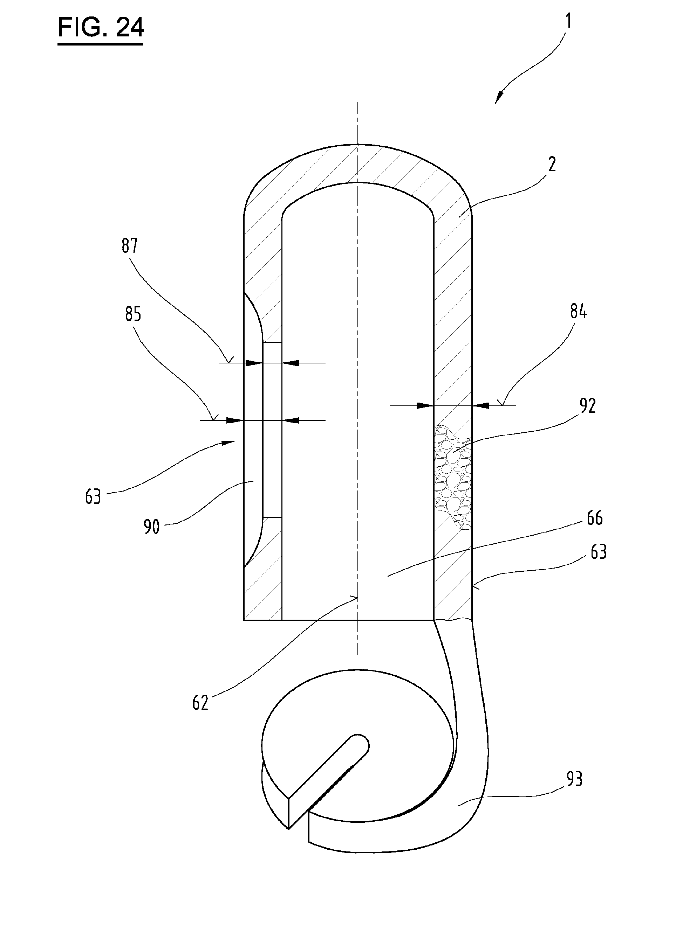

Dieser Kunststoffschaum weist bevorzugt einen grossen Teil offener Zellen auf, wie dies im Schnittbereich der Figur 24 schematisch angedeutet ist. Der Vorteil der Verwendung derartiger Kunststoffschäume liegt darin, dass diese bei Ausdehnung, beispielsweise durch die Vibrationen oder Vibrationserzeuger, die aus aufblasbaren Elementen bestehen, nach der Ausdehnung das Zusammenziehen in die Ausgangsposition in radialer Richtung unterstützen, also die selbsttätige, elastische Rückstellung aufgrund ihrer hohen Elastizitäten.

Bei Verwendung derartiger Kunststoffschäume ist es auch möglich, beispielsweise durch Temperatureinstellung bei der Herstellung der Saugkörper partiell die Aussenhaut zu verschliessen, das heisst im Aussenbereich aufgrund partieller Temperaturbeeinflussung eine geschlossene Haut zu erzielen. Dadurch kann auch ein Austritt von in der Schaumstruktur aufgenommener Flüssigkeit zusätzlich erschwert oder verhindert werden. Eine derartige Ausbildung kann vor allem im proximalen Endbereich 60 des Saugkörpers 2 von Vorteil sein.

Die Herstellung des Saugkörpers 2 kann im Rahmen der Erfindung aus unterschiedlichsten Materialien erfolgen, die unter den Bedingungen im Bereich der Vagina bzw. des Uterus, das heisst bei Körpertemperaturen, einen pH-Wert von ca. 4 über längere Zeit ihren Zustand aufrecht erhalten und keine Giftstoffe oder schleimhautschädlichen Lösungen oder dergleichen abgeben. Vor allem Polyurethanweichschäume mit sehr niedrigem Raumgewicht und einer überwiegend offenzelligen Struktur können mit Vorteilen eingesetzt werden.

Der Vorteil für derartige, schaumförmige Strukturen liegt auch darin, dass die offenen Zellen im Auslieferungszustand oder vor dem Einführen in die Vagina mit Medikamenten und/oder Gleitmitteln gefüllt werden können, die im eingesetzten Zustand an die Schleimhäute der Vagina bzw. des Uterus abgegeben werden können. Diese Abgabe kann durch die Vibrationswirkung der Vibrationserzeuger zusätzlich noch unterstützt werden.

Von Vorteil ist es weiterhin, wenn die verwendeten Materialien biologisch abbaubar sind und beispielsweise aus PLA oder sonstigen, biologisch abbaubaren Kunststoff oder Kunststoffgemischen, Fäden oder Fasern bestehen. Gleichermassen können auch Gehäuseteile, Stützelemente oder Führungselemente oder auch rotierende Teile des Vibrationserzeugers 3 und/oder eines Strömungserzeugers 72 gegebenenfalls aus biologisch abbaubaren Materialien gebildet sein, beispielsweise gepressten Fasern oder Fäden, biologisch abbaubaren Kunststoffen, wie PLA, oder recycelten Kunststoffe, wie R-PP, R-PET oder dergleichen, gegebenenfalls in unterschiedlichen Mischungen oder als mehrlagige Teile aus diesen Materialien.

Im dargestellten Beispiel gemäss den Figuren 1 und 2 enthält der Vibrationserzeuger 3 der Tamponanordnung 1 eine kleine Turbine 13 als Antrieb 73 für den Vibrationserzeuger 3, wie dies nachstehend unter Bezugnahme auf die Figur 2 näher beschrieben wird. Zum Betrieb der Turbine 13 wird dieser ein Fluid durch eine zweckmässig flexible Fluidleitung 5 zugeführt. Das andere Ende der Fluidleitung 5 ist mittels einer Kupplung 11 mit einer Energieversorgungseinheit 74 verbunden. Letztere umfasst im dargestellten Ausführungsbeispiel zur Beaufschlagung eines Strömungserzeugers 72, wie beispielsweise eines als Pumpe 8 ausgebildeten Druckerzeugers, eine Energiequelle 9, beispielsweise eine Stromquelle, die als Batterie oder Akkumulator ausgebildet sein kann. Die Pumpe 8 und/oder die Energiequelle 9 ist in einem Gehäuse 10 angeordnet und speist die Turbine 13 mit einem Fluid.

Eine Länge 75 der Fluidleitung 5 zwischen dem Strömungserzeuger 72 und dem Gehäuse 10 der Energieversorgungseinheit 74 ist vorteilhaft grösser als 20 mm, bevorzugt grösser als 50 mm. Diese Länge 75 wird üblicher Weise so festgelegt, dass die Energieversorgungseinheit 74 und/oder der Strömungserzeuger 72 an einem Kleidungsstück, z.B. einem Slip, oder einer Slipeinlage der Benutzerin der Tamponanordnung 1 befestigt werden kann und die Verbindung zum Saugkörper 2 spannungsfrei bzw. lose erfolgen kann.

Zudem enthält die Energieversorgungseinheit 74 bzw. der Strömungserzeuger 72 in diesem Beispiel einen Druckbehälter 7. Der Druckbehälter 7 kann eine beliebige Form haben, ist aber vorzugsweise als zylindrische Druckgaspatrone ausgebildet und besteht beispielsweise aus Metall oder Kunststoff.

Das Beispiel nach Figur 1 ist als offenes System dargestellt, bei dem als Fluid ein Gas oder Gasgemisch eingesetzt wird. Das vom Antrieb 73 des Vibrationserzeugers 3 kommende, entspannte Gas wird durch eine weitere Fluidleitung 6 abgeleitet, die vorteilhaft ausserhalb des Körpers der die Tamponanordnung 1 tragenden Person endet und das Gas in die Umgebung entlässt. Als Gas eignet sich jedes für die Haut und die Schleimhaut ungiftige Gas, wie beispielsweise Luft, CO₂ (Kohlendioxid) oder N₂O (Distickstoffmonoxid oder Stickoxydul).

In einer Abwandlung der Ausführungsart nach den Figuren 1 und 2 kann die Tamponanordnung 1 auch als geschlossenes System ausgebildet werden, indem die weitere Fluidleitung 6 nicht in der Umgebung mündet, sondern das entspannte Fluid zur Pumpe 8 zurückführt, vorzugsweise ebenfalls über die Kupplung 11. In diesem Fall kann das Fluid auch eine Flüssigkeit sein, zum Beispiel Wasser.

In einer weiteren Abwandlung der Ausführungsart nach den Figuren 1 und 2 kann auf die Pumpe 8 und die Energiequelle 9 verzichtet und der Druckbehälter 7 so dimensioniert sein, dass dessen Druck und Inhalt für den Betrieb des Antriebes 73 des Vibrationserzeugers 3 während einer gewünschten Zeit ausreicht. Für die Steuerung und Regelung des Vibrationserzeugers 3 kann mindestens ein hier nicht dargestelltes Ventil vorgesehen sein, mit dem die Menge und/oder der Druck des dem Antrieb 73 zugeführten Druckfluids, das in diesem Fall bevorzugt ein Gas oder Gasgemisch ist, veränderbar ist, um die Frequenz und/oder Intensität bzw. Amplitude der Vibration durch das Ventil zu verändern.

Dieses Ventil kann vorteilhaft in die Kupplung 11 integriert oder mit dieser verbunden sein oder werden. Die Kupplung 11 kann zum Beispiel eine durch ein Gewinde bewegbare Nadel, insbesondere Hohlnadel, enthalten, die zur Inbetriebnahme der Tamponanordnung 1 eine zum Beispiel am Druckbehälter 7 angeordnete Membrane durchdringt und damit den Gasfluss durch die Fluidleitung 5 ermöglicht. Bei jeder Art einer lösbaren Kupplung 11 ist es vorteilhaft, wenn diese mit einem Rückschlagventil (nicht dargestellt) bzw. einer beim Öffnen und Schliessen selbstverschliessenden Schnellverschlusskupplung verbunden ist, um einen Austritt von Gas aus dem Druckbehälter 7 zu verhindern, wenn dieser noch Druckgas enthält, wenn die Kupplung 11 gelöst wird.

Weiter ist eine Ausführungsart denkbar, bei welcher der Druckbehälter 7 im Auslieferungszustand leer ist und erst bei Bedarf durch einen Druckerzeuger gefüllt wird. Der Druckerzeuger kann dabei wie in Figur 1 dargestellt im Gehäuse 10 angeordnet sein. Er kann aber auch als stationäre, von der Tamponanordnung 1 getrennte Einheit ausgebildet sein, an welcher der Druckbehälter 7 bei Bedarf angekoppelt und aufgefüllt werden kann. Weiter muss der Strömungs- bzw. Druckerzeuger 72 nicht zwingend eine Pumpe sein, sondern es kann sich auch um Mittel handeln, die mit Hilfe eines chemischen Vorgangs Druck, insbesondere Gasdruck, erzeugen, beispielsweise eine kleine Sprengladung, wie man dies bei den Airbags in Transportmitteln kennt.

Figur 2 zeigt den Tampon von Figur 1 in einer vergrösserten Darstellung. Die erwähnte Turbine 13 ist im Gehäuse 4 aufgenommen. Druckfluid aus der ersten Fluidleitung 5 strömt in Richtung des Pfeils 12 durch die Turbine 13, welche eine Welle 14 in Richtung des Pfeils 16 rotierend antreibt. Auf der Welle 14 ist eine Masse 15 exzentrisch angeordnet, welche bei Rotation Vibrationen erzeugt. Gegebenenfalls kann zwischen der Welle 14 und der die Masse 15 tragenden Welle 14 ein Reduktionsgetriebe angeordnet sein.

Das in der Turbine 13 entspannte Fluid strömt in Richtung des Pfeils 18 durch einen Abgaskanal 17 und verlässt den tamponartigen Saugkörper 2 durch die weitere Fluidleitung 6.

Die weitere Fluidleitung 6 kann wie erwähnt an einer Stelle enden, die beim bestimmungsgemässen Tragen des Saugkörpers 2 ausserhalb des Körpers der Trägerin liegt. In diesem Fall kann das freie Ende der weiteren Fluidleitung 6 zur Reduzierung von durch das Druckfluid, das in diesem Falle ein Gas ist, verursachten Geräuschen mit einem Schalldämpfer 76 ausgestattet sein (siehe Figur 1). Die Fluidleitung 5 und die weitere Fluidleitung 6 sind vorzugsweise als flexible Schläuche ausgebildet. Sie können auch zu einem Schlauch mit zwei Kammern zusammengefasst sein, wie sie im Handel erhältlich sind. Jeder Schlauch kann auch als Spiralschlauch ausgeführt sein.

Im Gegensatz zu den Darstellungen nach Figur 1 und 2, bei denen der Vibrationserzeuger 3 in einem Hohlraum 66 im Inneren des Saugkörpers 2 angeordnet ist, zeigen die Figuren 3 und 4 beispielhaft mögliche Anordnungen des Vibrationserzeugers 3 am tamponartigen Saugkörper 2.

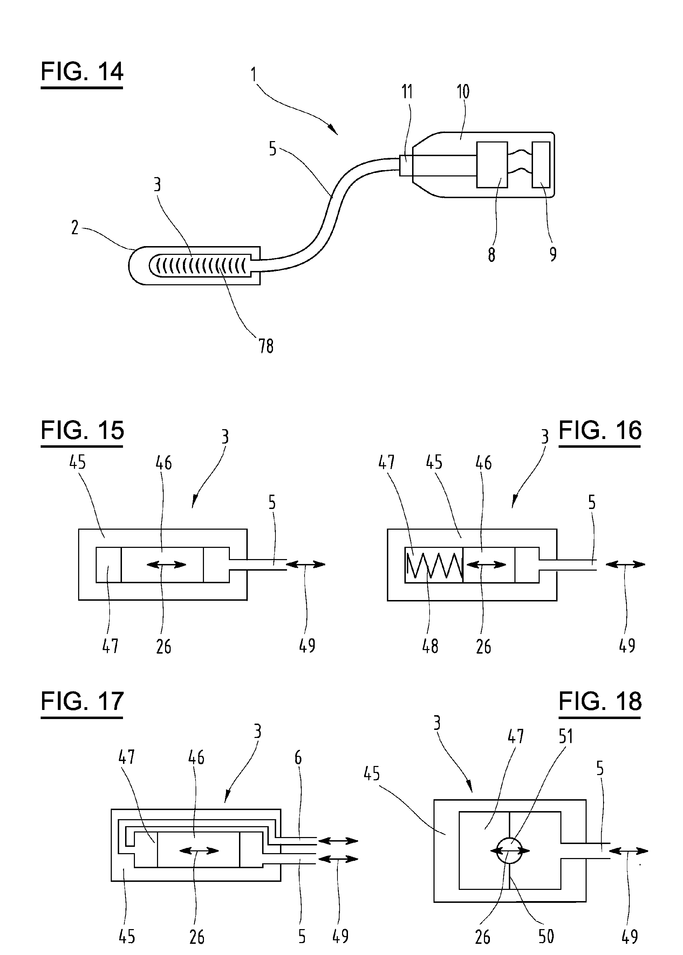

Bei dieser Ausführungsform ist der Saugkörper 2 mit einer Durchgangsöffnung für die Fluidleitung 5 versehen. Gemäss Figur 2 ist im Bereich des distalen Endes des Saugkörpers 2 ein als Vibrationserzeuger 3 wirkender, elastisch dehn- und selbstrückstellender, ballonartiger Expansionskörper gehalten bzw. abgestützt. Ausführungsarten eines derartigen Expansionskörpers sind in den Figuren 14, 19 und 20 illustriert. Das Ausdehnen und Zusammenziehen des Expansionskörpers kann durch bewusste positive und negative Fluidimpulse bzw. Druckimpulse, beispielsweise durch Beaufschlagen mit Druckfluid und Ablassen des Druckfluids, gegebenenfalls unter Aufbau eines Vakuums im Inneren des Expansionskörpers, erfolgen oder durch zugeführte Druckimpulse, wobei die Rückstellung und die Rückförderung des Fluids aus dem Expansionskörper 78 durch die Rückstellungskraft bei der elastisch selbsttätigen Rückstellung des Ballons des Expansionskörpers 78 in seine Ausgangsstellung erfolgen kann. Der Expansionskörper 78 stützt sich dabei auf das distale Ende 61 bzw. die Ausnehmung im Saugkörper 2 ab.

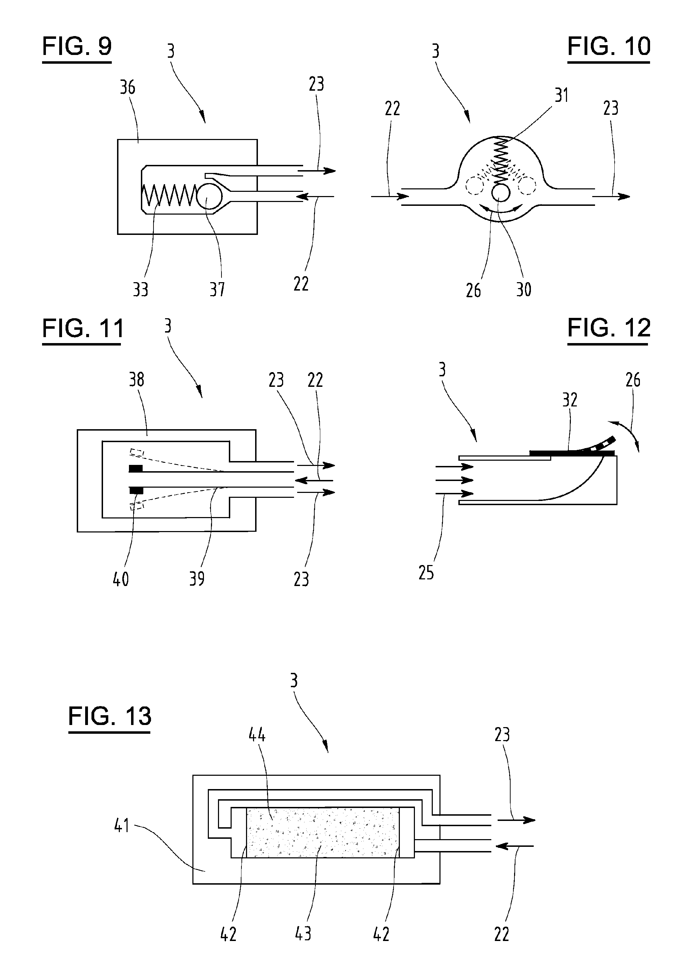

Figur 4 zeigt eine ähnliche Anordnung wie Figur 3, aber mit einem Vibrationserzeuger gemäss Figur 9.

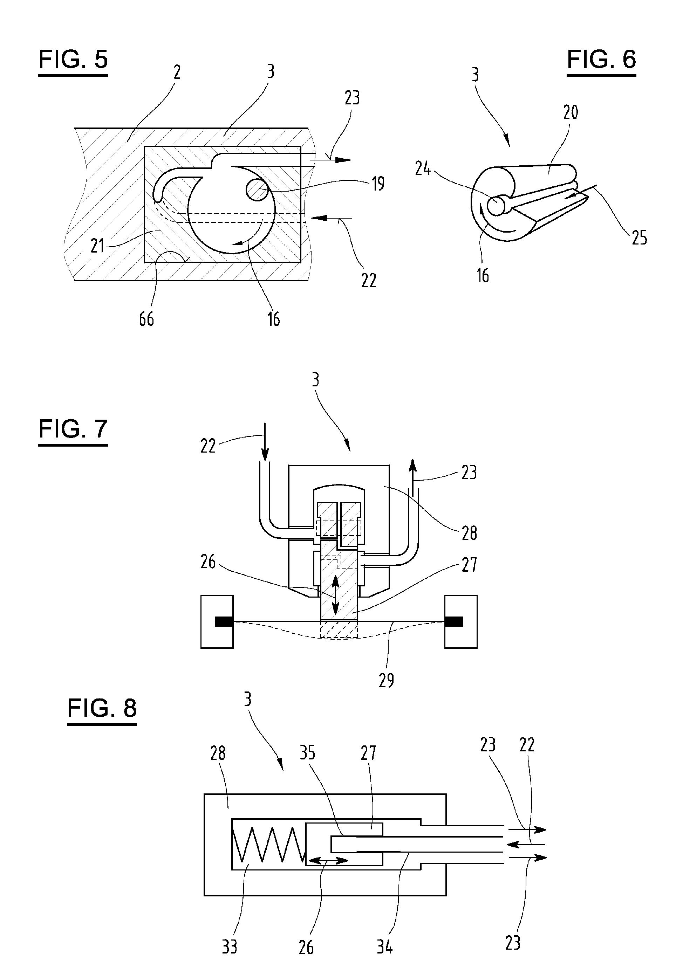

Figur 5 zeigt eine andere Ausführungsart des Vibrationserzeugers 3 in einem Hohlraum 66 eines Saugkörpers 2. In einem Gehäuse 21 sind ein Hohlraum und Fluidkanäle so angeordnet, dass ein im Hohlraum angeordneter Körper 19 durch einen in den Hohlraum eintretenden Fluidstrom (Pfeil 22) in Richtung des Pfeils 16 herumgewirbelt wird. Der Körper 19 kann beispielsweise eine Kugel oder ein Zylinder sein. Die bei der Bewegung des Körpers 19 entlang der Innenwand des Hohlraums auf das Gehäuse 21 übertragenen Kräfte bringen dieses zum Vibrieren. Das Fluid verlässt den Hohlraum in Richtung des Pfeils 23.

Bei dem in Figur 6 dargestellten Ausführungsbeispiel des Vibrationserzeugers 3 ist in dem Hohlraum des Gehäuses 21 gemäss Figur 5 ein nicht rotationssymmetrischer Körper 20 frei drehbar auf einer Achse 24 angeordnet. Wird der Körper 20 entsprechend den Pfeilen 25 von Druckfluid angeströmt, wird er gemäss Pfeil 16 in Rotation versetzt und erzeugt aufgrund seiner Unwucht Vibrationen. Die durch das Fluid angeströmte Fläche kann gegenüber der Achse 24 geneigt sein. Ein wesentlicher Vorteil dieser Ausführungsart besteht darin, dass sowohl der Antrieb als auch die Vibrationserzeugung von ein- und demselben Körper 20 übernommen wird.

Die in Figur 7 illustrierte Ausführungsart des Vibrationserzeugers 3, der in einen Hohlraum 66 eines Saugkörpers 2 eingebaut sein kann, basiert auf einer Anordnung aus einem in einem Zylinder 28 durch Fluiddruck verschiebbaren Kolben 27. Im Kolben 27 sind Kanäle derart angeordnet, dass in der in Figur 7 dargestellten Position des Kolbens 27 entsprechend dem Pfeil 22 einströmendes Fluid in den geschlossenen Raum oberhalb des Kolbens 27 geleitet wird, wodurch der Kolben 27 entgegen der Kraft einer Feder 29 abwärts gedrückt wird. Bei dieser Abwärtsbewegung des Kolbens 27 wird der das Druckfluid zuleitende Kanal verschlossen und ein das Druckfluid entsprechend dem Pfeil 23 ableitender Kanal geöffnet, so dass der Kolben 27 durch die Kraft der Feder 29 wieder nach oben verschoben wird. Somit kann sich der Kolben 27 entsprechend dem Doppelpfeil 26 auf- und ab bewegen und dadurch eine Vibration erzeugen.

Eine ähnliche Anordnung wie Figur 7 zeigt Figur 8, gemäss welcher ebenfalls ein Kolben 27 in einem Zylinder 28 entsprechend einem Doppelpfeil 26 hin- und her verschiebbar ist und dadurch Vibrationen erzeugt. Im Betrieb strömt Fluid in Richtung des Pfeils 22 durch ein Rohr 34, welches in einer Vertiefung 35 im Kolben 27 endet. Durch den Fluiddruck wird der Kolben 27 entgegen der Kraft einer Feder 33 in der Figur nach links bewegt, bis der Kolben 27 eine Position erreicht hat, in der das Rohr 34 nicht mehr in die Vertiefung 35 im Kolben 27 eintaucht. Das Fluid strömt nun am Kolben 27 vorbei in den Zylinder 28 und verlässt diesen in Richtung der Pfeile 23, so lange, bis der Kolben 27 wieder durch die Kraft der Feder 33 zum Rohr 34 hin verschoben wird. Durch rhythmische Wiederholung dieser Kolbenbewegung entstehen Vibrationen. Auch dieser Vibrationserzeuger 3 kann mit seinem Zylinder 28 in einen Hohlraum 66 des Saugkörpers 2 eingesetzt sein.

Es ist aber auch möglich, dass beispielsweise die Feder 33 direkt im Hohlraum 66 des Saugkörpers 2 angeordnet ist und sich auch der bewegliche Kolben 27 in diesem Hohlraum 66 bewegt. Anstelle einer dicken Zylinderwand des Zylinders 28 ist es aber auch möglich, mit einer sehr dünnen Aussenwand des Zylinders 28 die notwendige Druckfestigkeit für die Führung des beweglichen Kolbens 27 durch die Abstützung auf die verdichteten Bereiche des Saugkörpers 2 sicherzustellen.

In den nachfolgenden Figuren 9 bis 13 sind Vibrationserzeuger 3 gezeigt, die gegebenenfalls in einem Gehäuse jeweils in einen Hohlraum 66 eines Saugkörpers 2 der Tamponanordnung 1 eingesetzt werden können.

Figur 9 zeigt eine weitere Ausführungsart eines Vibrationserzeugers 3, bei dem ebenfalls ein Fluid entsprechend einem Pfeil 22 zugeführt wird und in Richtung eines Pfeils 23 abfliesst. Eine in einem Gehäuse 36 angeordnete Feder 33 spannt eine Kugel 37 in Richtung eines Sitzes vor, so dass die Kugel 37 den Zufluss von Fluid behindert. Durch steigenden Fluiddruck in der Zuleitung wird die Kugel 37 vom Sitz abgehoben, wodurch der Druck im Zufluss wieder sinkt. Diese Vorgänge wiederholen sich rhythmisch und erzeugen Vibrationen, die sich auf den Saugkörper 2 übertragen.

Figur 10 zeigt eine andere Ausführungsart des Vibrationserzeugers 3. Ein Körper 30 ist in einem Raum an einer Feder 31 befestigt. Wenn ein Fluid in Richtung des Pfeils 22 in den Raum strömt und den Raum in Richtung des Pfeils 23 wieder verlässt, bringt dies den Körper 30 in Richtung des Doppelpfeils 26 zum Schwingen, was an sich schon als Vibrationen spürbar ist. Bei stärkeren Ausschlägen stösst der Körper an die Innenwand des Raumes, wodurch die Vibrationen verstärkt werden.

Der Vibrationserzeuger 3 nach Figur 11 basiert auf einem flexiblen Rohr 39, das in einem Gehäuse 38 einseitig fest eingespannt ist und durch das Fluid entsprechend dem Pfeil 22 unter Druck zugeführt wird. Durch das ausströmende Fluid führt das freie Ende des Rohres 39 zufällige Bewegungen aus, so wie ein Wasserschlauch, der nicht festgehalten wird. Das Fluid verlässt das Gehäuse 38 in Richtung des Pfeils 23. Zur Verstärkung der durch die Bewegungen des freien Endes des flexiblen Rohres 39 verursachten Vibrationen kann das freie Ende des flexiblen Rohres 39 mit einer zusätzlichen Masse 40 ausgestattet sein. Die einzelnen Komponenten können auch so ausgestaltet sein, dass das freie Ende des flexiblen Rohres 39 und/oder die daran angeordnete Masse 40 bei den Bewegungen am Gehäuse 38 anschlagen und so die Vibrationsimpulse verstärken.

Figur 12 zeigt eine weitere Ausführungsart des Vibrationserzeugers 3, die sich insbesondere für den Betrieb mit einem Gas oder Gasgemisch eignet. Ein in einen Raum entsprechend den Pfeilen 25 einströmender Gasstrom bringt eine am Ausgang des Raumes angeordnete Lamelle 32 in Richtung des Doppelpfeils 26 zum Schwingen. Es ist auch möglich, die Lamelle 32 durch in umgekehrter Richtung strömendes Gas zum Schwingen zu bringen, ähnlich wie dies beispielsweise bei einem Holzblasinstrument geschieht. Die Vibrationen werden durch die Schwingungen der Lamelle 32 und deren Aufschlagen auf die den Raum begrenzende Wand erzeugt.

Figur 13 zeigt eine spezielle Ausführungsart eines Vibrationserzeugers 3. Im einem Gehäuse 41 ist ein mit einer Flüssigkeit 43 gefüllter Raum durch zwei Membranen 42 begrenzt, welche für Gas, aber nicht für Flüssigkeit durchlässig sind. Wird ein Gas entsprechend dem Pfeil 22 zugeführt, entstehen in der Flüssigkeit 43 Blasen 44, die Vibrationen verursachen, ähnlich wie dies von Sprudelbädern bekannt ist. Das Gas wird nach dem Passieren der Flüssigkeit 43 und der in der Figur links angeordneten Membrane 42 in Richtung des Pfeils 23 abgeführt.

Figur 14 zeigt in einer schematischen Darstellung eine weitere Ausführungsart einer Tamponanordnung 1. Im tamponartigen Saugkörper 2 ist ein sehr einfach aufgebauter Vibrationserzeuger 3 angeordnet, der aus einem ballonartigen, gummielastischen Expansionskörper 78 besteht und durch ein pulsierendes Fluid rhythmisch expandiert und kollabiert. Dazu wird nur eine einzige Fluidleitung 5 benötigt, die mit einer in einem Gehäuse 10 angeordneten Pumpe 8 verbunden ist, vorzugsweise über eine einfach lösbare Kupplung 11. Alternativ könnte die Kupplung 11 auch auf der Seite des Tampons 2 angeordnet sein. Die Pumpe 8 wird im dargestellten Beispiel durch eine Stromquelle 9 mit elektrischer Energie versorgt. Da es sich hier um ein geschlossenes System handelt, kann eine Flüssigkeit als Fluid eingesetzt werden. Eine Flüssigkeit hat zudem bei dieser Ausführungsart den Vorteil, dass sie auf Grund ihrer Inkompressibilität Druckstösse besser von der Pumpe 8 zum Vibrationserzeuger 3 überträgt als ein Gas.

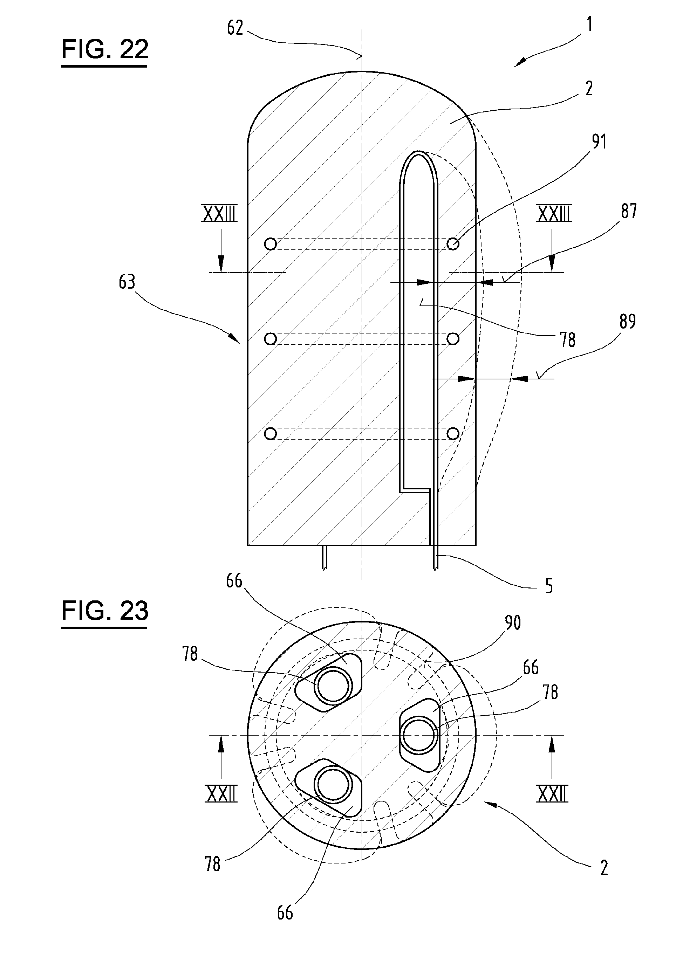

Zweckmässig ist es bei einigen tamponartigen Saugkörpern 2, wenn die Widerstandskraft gegen eine Verformung des Saugkörpers 2 in radialer Richtung geringer ist als die in radialer Richtung durch den Vibrationserzeuger 3 wirkende Expansionskraft. Die Rückstellung des Saugkörpers bzw. von Teilen des Saugkörpers, die durch die radiale Kraft verformt werden, kann durch eine durch den Vibrationserzeuger 3 bewirkte Rückstellkraft, z.B. durch Vakuumeinwirkung, erfolgen. Zweckmässig ist es hierbei, wenn eine Bewegungsverbindung zwischen dem Saugkörper 2 bzw. verstellbaren Teilen des Saugkörpers 2 besteht. Es ist aber ebenso möglich, wie nachstehend im Detail anhand der Ausführungsformen gemäss Figur 22 erläutert werden wird, den Saugkörper derart auszubilden, dass der Saugkörper 2 bzw. verstell- bzw. verformbare Teile des Saugkörpers 2 derart ausgebildet sind, dass sie eine elastische Selbstrückstellung bewirken.

Dies kann durch in den Saugkörper 2 eingearbeitete, diesen umhüllende bzw. mit diesem verbundene Rückstellelemente, wie beispielsweise Zugelemente 91 (Figur 22), elastische Fäden, Einlagen oder Umhüllungen, erreicht werden, die in radialer Richtung selbsttätig wirkende elastisch wirkende Rückstellkräfte bewirken. Diese Rückstellkräfte sind dann üblicherweise derart auszulegen, dass sie kleiner sind als die radialen Dehnkräfte des Vibrationserzeugers 3, aber grösser als der Widerstand des Vibrationserzeugers 3, insbesondere eines durch Fluid dehnbaren Expansionselementes bei der Rückstellung in die entspannte Ausgangsstellung.

Von Vorteil kann es hierbei sein, wenn die Rückstellkraft so bemessen ist, dass beispielsweise das in dem Expansionskörper vorhandene Fluid, insbesondere ein Gas wie etwa Luft, in die freie Atmosphäre oder in einen druckfreien Tank ausgetragen werden kann.

Drei weitere Ausführungsarten von Vibrationserzeugern 3 sind in den Figuren 15 bis 17 dargestellt und ebenfalls für die Verwendung in einer erfindungsgemässen Tamponanordnung 1 geeignet. Dazu ist zumindest ein Teil des Saukörpers 2 in den Hohlraum 66 einzubauen. Ein Kolben 46 wird in einem Zylinder 45 durch ein mittels einer Leitung 5 zugeführtes, gemäss dem Doppelpfeil 49 pulsierendes Fluid in eine hin- und her gehende Bewegung entsprechend dem Doppelpfeil 26 versetzt.

Bei der Ausführungsart nach Figur 15 ist auf der der Fluidleitung 5 abgewandten Seite des Kolbens 46 ein geschlossener Raum 47 angeordnet, der vorteilhaft mit einem Gas gefüllt ist, das auf den Kolben 46 wie eine Feder wirkt. Alternativ kann der Raum 47 gemäss Figur 16 eine auf den Kolben 46 einwirkende Feder 48 enthalten. In diesem Fall ist es vorteilhaft, wenn der Raum 47 durch eine kleine Öffnung (nicht dargestellt) mit der Umgebung verbunden ist, damit sich kein unerwünschtes Fluidpolster bildet.

Bei der Ausführungsart nach Figur 17 ist der Raum 47 mit der weiteren Fluidleitung 6 verbunden, welche wie weiter vorne beschrieben in der Umgebung enden oder zur Pumpe 8 zurückgeführt sein kann.

Bei der Ausführungsart nach Figur 18 ist an Stelle eines Kolbens eine den Raum 47 abtrennende, elastische Membrane 50 angeordnet. Diese trägt eine Zusatzmasse 51, um den Impuls der Vibrationen zu verstärken.

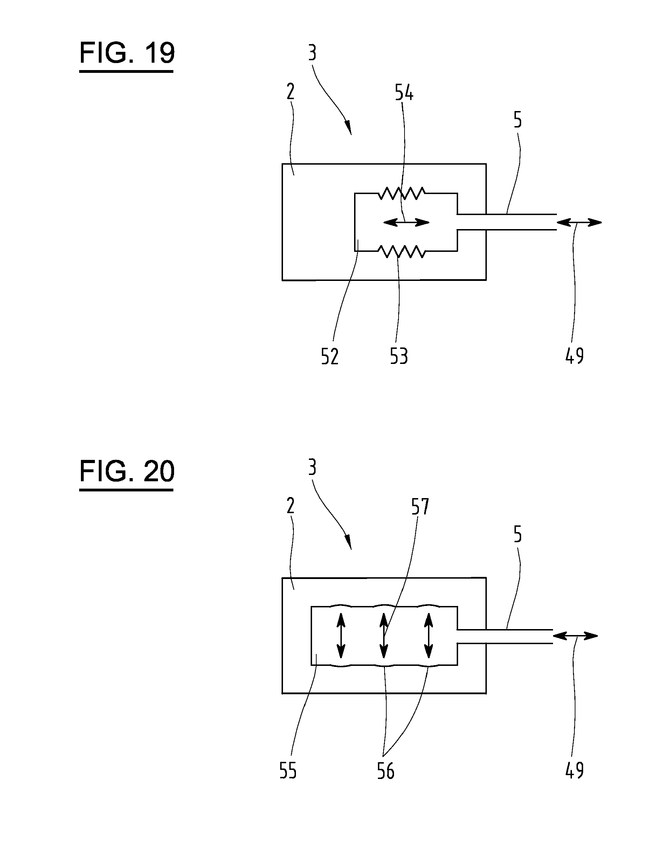

Die Vibrationserzeuger 3 nach den Figuren 19 und 20 basieren auf einem elastischen Hohlkörper 52 bzw. 55, der direkt in einem tamponartigen Saugkörper 2 eingebettet sein kann. Sie sind ebenfalls dazu vorgesehen, mit einem pulsierenden Fluiddruck entsprechend dem Doppelpfeil 49 beaufschlagt zu werden. Der Hohlkörper 52 nach Figur 19 hat einen Faltenbereich 53, der eine rhythmische Ausdehnung und Schrumpfung in Längsrichtung entsprechend dem Doppelpfeil 54 ausführt, wenn er mit einem Fluid mit pulsierendem Druck beaufschlagt wird. Der Hohlkörper 55 nach Figur 20 hat an seinem Umfang Wandungsbereiche 56 mit reduzierter Wandstärke, so dass sich bei Beaufschlagung mit einem Fluiddruck diese Bereiche 56 in Richtung der Doppelpfeile 57 ausdehnen und zusammenziehen.

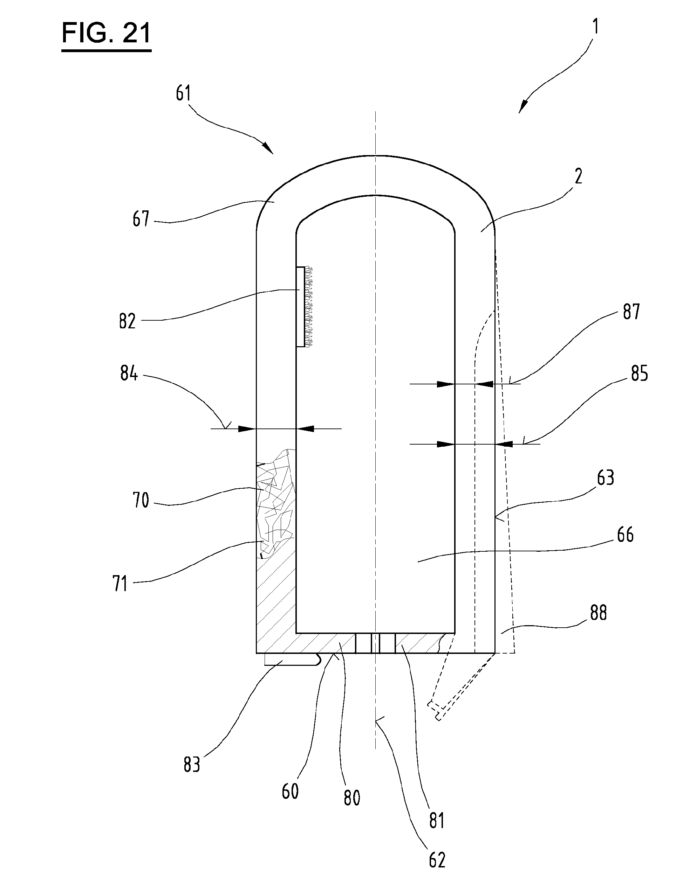

Eine weitere Ausführungsvariante ist in Figur 21 gezeigt. Bei dieser Ausführungsvariante ist der Saugkörper 2 der Tamponanordnung 1 haubenförmig ausgebildet und ist in seinem distalen Ende 61 mit einer Stirnwand 67 kuppelartig ausgebildet, beispielsweise in Form einer Kugelkalotte oder eines kegelförmigen Abschnitts mit abgerundeter Spitze. Im gegenüberliegenden Bereich des proximalen Endes 60 kann der Saugkörper 2 mit in Richtung seiner Längsachse vorragenden Verschlusslappen 80, 81 versehen sein, die so weit nach aussen verformt werden können, dass sie etwa in Längsrichtung der Längswand des als Hohlzylinder ausgebildeten mittleren Teils 63 des Saugkörpers 2 verlaufen, wie dies mit strichlierten Linien angedeutet ist. Dadurch wird in dem Saugkörper 2 ein Hohlraum 66 geschaffen, der über die Verschlusslappen 80 und 81 von aussen zugänglich ist.

Damit kann in diesem Hohlraum 66 ein Vibrationserzeuger 3 gegebenenfalls gemeinsam mit einem Strömungserzeuger 72 eingeführt und darin fixiert werden. Diese Fixierung kann über am Gehäuse des Vibrationserzeugers 3 und/oder des Strömungserzeugers 72 angeordnete Vorsprünge, Verankerungsorgane oder, wie schematisch in einem Teilbereich des Hohlraums 66 angedeutet, durch ein Klettband 82, aber auch durch Verkleben erfolgen. Gegebenenfalls ist es auch ausreichend, wenn die proximale Stirnseite 60, wie im Bereich des Verschlusslappens 80 angedeutet, nach dem Einsetzen des Vibrationserzeugers 3 und/oder des Strömungserzeugers 42 mit einer Scheibe 83, beispielsweise aus einer Folie, zur Gänze verschlossen wird, sodass auch eine Halterung der im Hohlraum 66 angeordneten Bauteile sichergestellt ist.

Diese Scheibe 83, die eine Durchgangsöffnung für zumindest eine Leitung 5, 6 aufweisen kann, kann beispielsweise aus einer Aluminiumfolie, einem Gewebe- oder Gewirketeil, einem Strickteil oder einer Kunststofffolie gebildet sein, die mit der Stirnseite 60 durch Kleben, Heissversiegeln oder dergleichen verbunden sein kann.

Im vorliegenden Fall weist der Saugkörper 2 in seinem mittleren Teil 63 eine gleiche Wandstärke 84, 85 auf. Die Wandstärken 84, 85 können jedoch über den Umfang verteilt variieren, um unterschiedliche Verformungen des Saugkörpers 2 zu ermöglichen. Der Saugkörper 2 besteht im vorliegenden Fall aus Fäden und/oder Fasern 70, 71, wie in einem Teil schematisch dargestellt.

Bei der Aufnahme eines Vibrationserzeugers 3, wie beispielsweise anhand der vorstehenden Figuren 1 und 2 sowie 5 bis 13 beschrieben ist, ist es vorteilhaft, wenn der Saugkörper 2 sehr hoch verdichtet ist und eine relativ geringe elastische Verformung in radialer Richtung bzw. der Wandstärken 84, 85 während der mit dem Vibrationserzeuger 3 erzeugten Vibrationen ermöglicht. Damit werden die mit dem Vibrationserzeuger 3 erzeugten Schwingungen mit relativ geringem Amplitudenverlust in die benachbarten Körperteile der Benutzerin übertragen.

Vorteilhaft ist es vor allem, wenn die elastische Verformbarkeit des Saugkörpers 2 im mittleren Teil 63 hinsichtlich einer Verringerung der Wandstärke einen höheren Kraftaufwand erfordert als die mit dem Vibrationserzeuger 3 erzeugte radiale Kraftkomponente. Gegebenenfalls kann es aber auch von Vorteil sein, dass die durch den Vibrationserzeuger 3 radiale Kraft etwas grösser ist als die Verformungskraft in Richtung der Wandstärke 84 und 85 des Saugkörpers 2, da dadurch über einen gewissen Bereich unter Umständen eine elastische Rückstellung und Bewegung der äusseren Oberfläche und der äusseren Schichten des Saugkörpers 3 gegenüber den benachbarten Körperteilen erzielt werden kann. Dies kann zur Abgabe von in den Oberflächenbereichen des Saugkörpers 2 angeordneten Medikamenten, Gelen oder Flüssigkeiten oder auch zur Verbesserung der Aufnahme von Körperflüssigkeiten aus den unmittelbar benachbarten Körperteilen bzw. dem Nachfördern von Körperflüssigkeiten aus dem Körperinneren in den Bereich des Saugkörpers 2 behilflich sein.

Eine Ausführungsvariante der vorliegenden Ausführungsform kann auch vorsehen, dass dann, wenn die radialen Kräfte des Vibrationserzeugers 3 beispielsweise nahe dem proximalen Ende 60 angeordnet sind, wie mit strichlierten Linien angedeutet, beispielsweise über den Umfang des Saugkörpers 2 verteilt mehrere in Richtung der Längsachse 62 verlaufende Schwächungsbereiche vorgesehen sind, in welchen also eine Wandstärke 87 geringer ist als in den benachbarten Bereichen. Dadurch ist es möglich, dass der Saugkörper 2 in Richtung seines proximalen Endes 60 elastischer ist, und beispielsweise während der Schwingungsbewegung die einzelnen Elemente des Saugkörpers 2, wie mit strichlierten Linien ebenfalls angedeutet ausweichen und vibrieren können, um somit Vibrationen mit einer grösseren Amplitude in den Körper zu übertragen. Dieses Auswärtsschwenken der Seitenwände des mittleren zylinderförmigen Teils 63 des Saugkörpers 2 vereinfacht auch den Einbau des Vibrationserzeugers 3 bzw. des Strömungserzeugers 72. In diesem Fall kann es von Vorteil sein, die Scheibe zum Verschliessen des Hohlraumes 66 wegzulassen oder aus einem sehr leicht dehnbaren Material herzustellen.

In den Figuren 22 und 23 ist eine weitere Ausführungsform der erfindungsgemässen Tamponanordnung gezeigt. In dem Saugkörper 2 sind über den Umfang mehrere Hohlräume 66 angeordnet, die sich in Richtung der Längsachse 62 der Tamponanordnung 1 rohrförmig erstrecken. Die Hohlräume 66 sind zwischen der Längsachse 62 und der Aussenseite des mittleren Teils 63 des Saugkörpers 2 angeordnet. Die Hohlräume 66 können auch zu einem gemeinsamen Hohlraum miteinander verbunden sein. In jedem dieser Hohlräume 66 stützen sich schlauchförmige Expansionskörper 78 auf einen stark verdichteten Innenteil des Saugkörpers 2 oder gegeneinander ab.

In diesem Fall werden die in diesen sehr schlanken Hohlräumen 66 eingebrachten, schlauchförmigen Expansionskörper 78 mit pulsierendem Fluid beaufschlagt. Hierfür kann jeder Hohlraum 66 separat über eine Fluidleitung 5 mit dem Fluid versorgt werden. Alternativ dazu können die Hohlräume 66 unmittelbar aufeinanderfolgend oder in unterschiedlicher Aufeinanderfolge von dem Fluid durchströmt werden, z.B. auch teilweise überlappend. Jeweils bei dem Einpressen von Fluid dehnt sich der Expansionskörper 78 aus. Hierdurch werden die Bereiche des Saugkörpers 2 mit geringerer Wandstärke 87, wie mit strichlierten Linien angedeutet, um eine Distanz 89 nach aussen verformt.

Diese Verformung kann auch dadurch unterstützt werden, dass benachbart zu den Hohlräumen 66, wie in der Draufsicht in Figur 23 gezeigt, Schwächungsbereiche bzw. Rillen oder Nuten 90 angeordnet sind. Die Rückstellung dieser Elemente bzw. der Wandteile des Saugkörpers 2 kann durch entsprechendes Anlegen eines ausreichenden Unterdrucks über die Fluidleitung 5 erfolgen. Alternativ kann die Rückstellung, wie ebenfalls durch strichlierte Linien angedeutet, durch elastische Zugelemente 91, beispielsweise eingearbeitete Gummifäden oder -ringe oder sonstige elastische Zugmittel erfolgen. Dadurch ist es nur notwendig, die Expansionskörper 78 mit Druckmittelstössen zu beaufschlagen, wogegen das Zusammendrücken der Expansionskörper 78 und das Auspressen des Fluids aus deren Innenraum durch die Zugelemente 91 durch eine selbsttätige elastische Rückstellung der Saugkörper 2 erfolgt. Unter anderem ist es beispielsweise auch möglich, dass in jenen Bereichen, in denen keine Rillen oder Nuten 90 bzw. Bereiche mit geringerer Wandstärke 87 vorgesehen sind, diese Bereiche des Saugkörpers 2 besonders stark zu verdichten, um dort eine hohe Aufnahmefähigkeit an Flüssigkeit einerseits und eine hohe Steifheit zu erreichen, sodass eine gerichtete Ausdehnung der flexiblen Teile bzw. Bereiche des Saugkörpers 2 erfolgen kann.

In Figur 24 ist eine Ausführungsvariante gezeigt, bei der der Saugkörper 2 der Tamponanordnung 1 wie in einem Teil des schraffierten Bereiches angedeutet, durch einen offenzelligen Schaum, insbesondere einen offenzelligen Kunststoffschaum 92, oder einen schaumartigen Gummikörper gebildet ist. In diesem Fall kann der im Inneren des Saugkörpers 2 vorgesehene Hohlraum 66 mit einer direkt an den mittleren Teil 63 des Saugkörpers 2 angeformten Verschlusskappe 93 nach dem Einbringen des Vibrationserzeugers 3 bzw. des Strömungserzeugers 72 verschlossen werden. Zum Hindurchführen zumindest einer Leitung 6 ist ein Schlitz in der Verschlusskappe 93 angeordnet.

Des Weiteren ist in diesem Ausführungsbeispiel gezeigt, dass bei Verwendung eines Expansionskörpers 78 als Vibrationserzeuger 3 zusätzlich zur Schwächung der Wandstärke in zumindest einem, bevorzugt mehreren, distanziert über den Umfang des Saugkörpers 2 verteilten Bereichen die Wandstärke 84, 85 auf eine geringere Wandstärke 87 verringert sein kann. Dies kann durch partielles Ausbildung von über einen Teilbereich der Dicke des Saugkörpers 2 von aussen in Richtung der Längsachse 62 vorragend ausgebildete Nuten 90 erfolgen. Zudem kann es zur Erzielung einer höheren Elastizität auch der hohlzylinderförmige mittlere Teil 63 durchtrennt bzw. durchschnitten sein, um eine noch höhere Flexibilität zu erreichen.

Durch die Verwendung eines schaumförmigen Saugkörpers 2 kann vorteilhafterweise eine höhere Feuchtigkeitsaufnahme erzielt werden. Zudem kann eine höhere Ansaugwirkung durch die pulsierende Beaufschlagung des Schaums erzielt werden. Selbstverständlich ist es aber auch möglich, die Saugkörper 2 gemäss den Figuren 21 bis 24 mit den Vibrationserzeugern 3 gemäss den Figuren 1, 2 und 5 bis 13 sowie 15 bis 18 zu betreiben. Vorteilhaft ist jedoch auch deren Verwendung in Verbindung mit den Vibrationserzeugern 3, wie sie in den Figuren 14 und 19 bzw. 20 dargestellt sind.

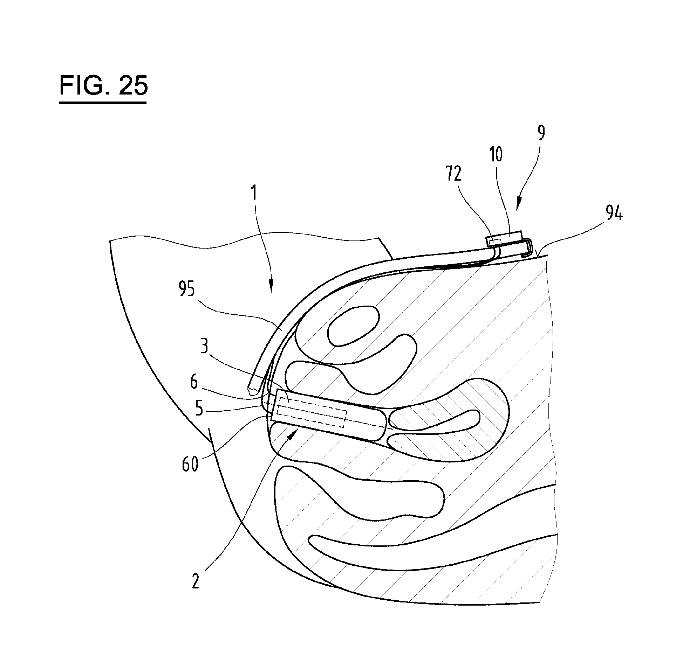

In Figur 25 ist schematisch die Anordnung einer Tamponanordnung 1 im Bereich der Vagina gezeigt. Die vom proximalen Ende 60 der Tamponanordnung 1 wegführenden Fluidleitungen 5 und 6 sind schematisch angedeutet und verlaufen im vorliegenden Beispiel zwischen einer Körperoberfläche 94 und einem Bekleidungsteil, beispielsweise einem Slip 95. Selbstverständlich ist es auch möglich, dass die Fluidleitung 5 oder die Fluidleitung 5, 6 in den Slip 95 eingearbeitet oder auf der von der Körperoberfläche 94 abgewendeten Seite des Slips 95 geführt werden. Dazu können auch entsprechende Öffnungen oder Durchbrüche im Slip 95 vorgesehen sein.

Am Slip 95, vorteilhafterweise im Bereich eines Gummizuges oder an einem Stoffteil des Slips 95, kann beispielsweise mit Klettbändern oder mit sonstigen Verschluss- oder Schnappelementen ein Gehäuse 10 befestigt sein, in dem die Energieversorgungseinheit 74 und gegebenenfalls der Strömungserzeuger 72 angeordnet sein können. Daraus ist zu ersehen, dass die Länge 75 der Fluidleitungen 5, 6 auf die entsprechenden Bekleidungsstücke, insbesondere die Befestigungsart des Gehäuses 10 mit der Energiequelle 9 bzw. dem Strömungserzeuger 72, abzustellen ist. Dazu kann es für unterschiedliche Verwendungen vorteilhaft sein, die Fluidleitungen 5, 6 als hochflexible Spiralleitungen auszuführen. Andererseits ist es aber auch möglich, die Bekleidungsstücke schon entsprechend mit Fluidleitungen 5, 6 auszustatten, sodass nur mehr die Energiequelle 9 und mit dem Gehäuse 10 gegebenenfalls gemeinsam mit dem Strömungserzeuger 72 einerseits und am anderen Ende des Saugkörpers 2 mit dem Vibrationserzeuger 3 anzuschliessen ist.

Daher kann die Länge der Fluidleitung bzw. Fluidleitungen 5, 6 zwischen 20 mm und 150 mm, bevorzugt zwischen 20 mm und 100 mm betragen - je nach Konfektionsgrösse des Bekleidungsstücks, insbesondere des Slips 95.

Vorteilhaft ist es, wenn die Fluidleitungen zwar in radialer Richtung formstabil sind, jedoch in Längsrichtung dehnbar. Damit kann eine Anpassung auf die unterschiedlich gewünschten Längen jeweils einfach erfolgen.

Die einzelnen in den Figuren gezeigten Ausführungen können auch den Gegenstand von eigenständigen, erfindungsgemässen Lösungen bilden. Die diesbezüglichen, erfindungsgemässen Aufgaben und Lösungen sind den Detailbeschreibungen dieser Figuren zu entnehmen.

Die Ausführungsbeispiele zeigen mögliche Ausführungsvarianten der erfindungsgemässen Tamponanordnung, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvarianten möglich sind, vom Schutzumfang mit umfasst.

### Bezugszeichenliste

- 1: Tamponanordnung
- 2: Saugkörper
- 3: Vibrationserzeuger
- 4: Gehäuse
- 5: Fluidleitung
- 6: weitere Fluidleitung
- 7: Druckbehälter
- 8: Pumpe
- 9: Energiequelle
- 10: Gehäuse
- 11: Kupplung
- 12: Pfeil
- 13: Turbine
- 14: Welle
- 15: Masse
- 16: Pfeil
- 17: Abgaskanal
- 18: Pfeil
- 19: Körper
- 20: Körper
- 21: Gehäuse
- 22: Pfeil
- 23: Pfeil
- 24: Achse
- 25: Pfeil
- 26: Doppelpfeil (Körper)
- 27: Kolben
- 28: Zylinder
- 29: Feder
- 30: Körper
- 31: Feder
- 32: Lamelle
- 33: Feder
- 34: Rohr
- 35: Vertiefung
- 36: Gehäuse
- 37: Kugel
- 38: Gehäuse
- 39: flexibles Rohr
- 40: Masse
- 41: Gehäuse
- 42: Membrane
- 43: Flüssigkeit
- 44: Blasen
- 45: Zylinder
- 46: Kolben
- 47: Raum
- 48: Feder
- 49: Doppelpfeil (Fluid)
- 50: Membrane
- 51: Zusatzmasse
- 52: Hohlkörper
- 53: Bereich von 52
- 54: Doppelpfeil
- 55: Hohlkörper
- 56: Bereiche von 55
- 57: Doppelpfeil
- 58:
- 59:
- 60: Proximales Ende
- 61: Distales Ende
- 62: Längsachse
- 63: Mittlerer Teil
- 64: Aussendurchmesser
- 65: Entnahmeorgan
- 66: Hohlraum
- 67: Stirnwand
- 68: Stirnwand
- 69: Einsatzteil
- 70: Fasern
- 71: Faden
- 72: Strömungserzeuger
- 73: Antrieb
- 74: Energieversorgungseinheit
- 75: Länge
- 76: Schalldämpfer
- 77:
- 78: Expansionskörper
- 79: Pfeil
- 80: Verschlusslappen
- 81: Verschlusslappen
- 82: Klettband
- 83: Scheibe
- 84: Wandstärke
- 85: Wandstärke
- 86:
- 87: Wandstärke
- 88:
- 89: Distanz
- 90: Nut
- 91: Zugelement
- 92: Kunststoffschaum
- 93: Verschlusskappe
- 94: Körperoberfläche
- 95: Slip

## Patentansprüche

1. Tamponanordnung (1) mit einem Saugkörper (2) und einem damit verbundenen Vibrationserzeuger (3), **dadurch gekennzeichnet, dass** die Tamponanordnung (1) einen Strömungserzeuger (8) für ein Fluid aufweist, welcher über eine Fluidleitung (5) mit dem Vibrationserzeuger (3) wirkverbunden oder wirkverbindbar ist.

2. Tamponanordnung (1) nach Anspruch 1, **dadurch gekennzeichnet, dass** der Strömungserzeuger (8) einen Fluiddruckerzeuger umfasst.

3. Tamponanordnung (1) nach Anspruch 2, **dadurch gekennzeichnet, dass** der Fluiddruckerzeuger einen mit Druckfluid vorbefüllten Druckbehälter (7), z.B. eine Druckgaspatrone, enthält oder daraus besteht.

4. Tamponanordnung nach einem der Ansprüche 2 bis 3, **dadurch gekennzeichnet, dass** der Fluiddruckerzeuger einen Druckbehälter (7) umfasst.

5. Tamponanordnung (1) nach einem der Ansprüche 2 bis 4,
**dadurch gekennzeichnet, dass** der Fluiddruckerzeuger und/oder der Druckbehälter (7) über eine Schnellverschlusskupplung mit dem Strömungserzeuger (8) und/oder mit dem Vibrationserzeuger (3) kuppelbar ist.

6. Tamponanordnung (1) nach einem der Ansprüche 2 bis 5,
**dadurch gekennzeichnet, dass** zwischen dem Fluiddruckerzeuger und dem Druckbehälter (7) ein bevorzugt einstellbares Druckreduzier- und/oder Überdruckventil angeordnet ist.

7. Tamponanordnung (1) nach einem der vorangehenden Ansprüche, **dadurch gekennzeichnet, dass** am Vibrationserzeuger (3) eine weitere Fluidleitung (6) angeschlossen ist.

8. Tamponanordnung (1) nach einem der vorangehenden Ansprüche, **dadurch gekennzeichnet, dass** die Fluidleitung (5) an dem mit dem Strömungserzeuger (8) verbundenen Ende und/oder an dem mit dem Vibrationserzeuger (3) verbundenen Ende mit lösbaren Kupplungsmitteln (11) ausgestattet ist.

9. Tamponanordnung (1) nach einem der vorangehenden Ansprüche, **dadurch gekennzeichnet, dass** am Vibrationserzeuger (3) und/oder am Saugkörper (2) zumindest ein bevorzugt flexibles Entnahmeorgan, insbesondere ein Zugelement, wie ein Faden oder eine Schnur, befestigt ist.

10. Tamponanordnung (1) nach einem der vorangehenden Ansprüche, **dadurch gekennzeichnet, dass** der Saugkörper (2) und der Vibrationserzeuger (3) und/oder der Strömungserzeuger (8) mit zusammenwirkenden Kupplungsteilen versehen sind.

11. Tamponanordnung (1) nach Anspruch 10, **dadurch gekennzeichnet, dass** der Kupplungsteil des Saugkörpers (2) in einem vom Saugkörper (2) zumindest zum Teil umschlossenen Hohlraum angeordnet ist.

12. Verwendung einer Tamponanordnung (1) nach einem oder mehreren der Ansprüche 1-11 zum Aufsaugen von Körperflüssigkeiten in einer Vagina und/oder zur Schwingungsbeaufschlagung eines weiblichen Körpers im Bereich der Vagina, **dadurch gekennzeichnet, dass** der mit dem Vibrationserzeuger (3) verbundene Saugkörper (2) in die Vagina eingesetzt wird.

13. Verfahren zum Aufsaugen von Körperflüssigkeiten in einer Vagina und/oder zur Schwingungsbeaufschlagung eines weiblichen Körpers im Bereich der Vagina mit einem in die Vagina eingesetzten Saugkörper (2) und einem mit diesem vibrationsverbundenen Vibrationserzeuger (3), wobei der Vibrationserzeuger (3) mit einem Strömungserzeuger (72) verbunden wird.

14. Verwendung eines Druckbehälters (7), insbesondere einer Druckgaspatrone, als Strömungserzeuger (8) in einer Tamponanordnung (1), insbesondere in einer Tamponanordnung (1) gemäss einem der Ansprüche 1 bis 11.
